# FASCICULE DE BREVET EUROPEEN

(11) **EP 3 600 369 B1**
(45) Date de publication et mention de la délivrance du brevet: **20.05.2026**
(21) Numéro de dépôt: 18711129.9
(22) Date de dépôt: 20.03.2018
(51) Int. Cl.: A61K 36/48, A61P 17/08, A61P 17/14

(54) **UTILISATION D'UN EXTRAIT DE COPAIFERA CONTRE L'ALOPECIE**
VERWENDUNG EINES COPAIFERA EXTRAKTES GEGEN ALOPECIA
USE OF AN EXTRACT OF COPAIFERA AGAINST ALOPECIA

(30) Priorité: 20.03.2017 FR 1752281
(43) Date de publication de la demande: 05.02.2020
(73) Titulaire: Pierre Fabre Dermo-Cosmétique, 81500 Lavaur (FR)
(72) Inventeur: FIORINI-PUYBARET, Christel, 31500 Toulouse (FR)
(74) Mandataire: Regimbeau
(86) Numéro de dépôt international: PCT/EP2018/057075
(87) Numéro de publication internationale: WO 2018/172379

(56) Documents cités:
- WO-A1-2013/084163
- FR-A1- 2 816 843
- KR-A- 20000 042 508
- DATABASE WPI Week 200923, Derwent World Patents Index; AN 2009-E69339, XP002776851
- DATABASE WPI Week 201707, Derwent World Patents Index; AN 2016-72170C, XP002776855
- "Prima fleur recommends treating skin to nourishing vegetable oil and essential oil serums to target any skin condition", MASSAGE MAGAZINE, 27 July 2011 (2011-07-27), pages 1 - 3, XP002776853, Retrieved from the Internet <URL:https://www.massagemag.com/prima-fleur-recommends-treating-skin-to-nourishing-vegetable-oil-and-essential-oil-serums-to-target-any-skin-condition-9515/> [retrieved on 20171219]
- VALDIR F. VEIGA ET AL: "Phytochemical and antioedematogenic studies of commercial copaiba oils available in Brazil", PHYTOTHERAPY RESEARCH., vol. 15, no. 6, 1 January 2001 (2001-01-01), GB, pages 476 - 480, XP055435217, ISSN: 0951-418X, DOI: 10.1002/ptr.976
- LIDIAM MAIA LEANDRO ET AL: "Chemistry and Biological Activities of Terpenoids from Copaiba (Copaifera spp.) Oleoresins", MOLECULES ONLINE, vol. 17, no. 12, 30 December 2012 (2012-12-30), DE, pages 3866 - 3889, XP055313075, ISSN: 1433-1373, DOI: 10.3390/molecules17043866

## Description

La présente divulgation concerne l'utilisation d'un extrait de *Copaifera* en cosmétique et dermatologie dans le traitement et/ou la prévention de l'alopécie ainsi que dans le traitement de la séborrhée.

### ART ANTERIEUR

Le genre *Copaifera* compte 35 espèces, toutes des arbres d'Amérique tropicale, c'est-à-dire au Mexique, le nord de l'Argentine et principalement au Brésil. Sur le territoire on dénombre plus de vingt espèces, les plus abondantes étant *C. officinalis, C. reticulta, C. multijuga. Copaifeira officinalis* est un arbre que l'on retrouve essentiellement au Brésil, en Colombie et au Venezuela. C'est un arbre d'une hauteur jusqu'à 25 m, à bois brun-rougeâtre. Les feuilles sont composées, paripennées avec 2 à 10 folioles, alternes ou sub-opposées, apiculées et inégalement arrondies à la base. Elles sont longues de 3 à 8 cm et larges de 2 à 4 cm. Les fleurs blanches, généralement sessiles sont groupées en inflorescences de 7 à 14 cm. Les fruits sont des petites gousses renflées à maturité de diamètre 20-25 mm glabre, apiculées renfermant une graine ovoïde.

L'oléorésine est une substance obtenue à l'aide d'incisions de l'écorce de plusieurs espèces de Copaifera. Localisée dans des canaux sécréteurs anastomosés du bois secondaire et de la moelle, son extraction nécessite donc des incisions très profondes du tronc ce qui lui permet de s'écouler naturellement de l'arbre.

Après distillation à la vapeur, ou hydrodistillation, l'oléorésine permet d'obtenir l'huile essentielle de Copaïba réputée dans la parfumerie.

L'oléorésine de Copaïba a été utilisée en médecine à partir du XVI^{ème} siècle par les indigènes du Brésil. Elle a une longue histoire d'utilisation dans la médecine traditionnelle brésilienne, pour soigner les blessures et faire disparaitre les cicatrices, fébrifuge, antiseptique des voies urinaires, contre la leucorrhée et la blennorragie. Considéré comme un tonique général, ses indications étaient les suivantes : maladie vénérienne, maladie respiratoire, asthme, rhumatisme, lésions dermiques secondaires, ulcères. A dose faible, c'est un stimulant d'action directe sur l'estomac. L'oléorésine de Copaïba diminue la sécrétion excessive de mucus provoquée par des inflammations. Aujourd'hui, l'oléorésine Copaïba est vendue en gélules dans les pharmacies du Brésil où elle est indiquée pour tous les types d'inflammations internes et les ulcères de l'estomac. En application locale, c'est un cicatrisant puissant à la fois antiseptique et anti-inflammatoire, il aide à la cicatrisation des plaies les plus difficiles. L'oléorésine de Copaïba serait très efficace sur les douleurs articulaires, les entorses bénignes, les hématomes, les tendinites. L'oléorésine est appliquée directement sur la peau. L'oléorésine est également utilisée sous forme d'huile de massage pour les muscles et les articulations douloureuses ou enflammées.

L'oléorésine de Copaifera, distillée ou non, est également utilisée en cosmétique, dans la fabrication de savons, de bains moussants, détergents et crèmes, et comme fixateur en parfumerie. L'oléorésine est parfois utilisée comme aromatisant dans le domaine alimentaire. L'oléorésine de Copaïba est également utilisée comme matériau d'artiste, en particulier dans les recettes de peinture à l'huile et dans la céramique de décoration.

L'oléorésine est un liquide incolore et peu consistant, il acquiert une consistance oléagineuse et une couleur jaune verdâtre avec le temps. Sa consistance et sa couleur varient un peu, suivant l'arbre dont il provient et suivant l'huile essentielle qui s'y trouve. Son odeur est forte et désagréable, sa saveur est amère et âcre. L'oléorésine est insoluble dans l'eau, soluble en totalité dans l'alcool et dans l'éther.

L'oléorésine de Copaifera *officinalis* est constituée de 2 fractions qui se distinguent par leur volatilité ; chacune des fractions étant caractérisée par des composés chimiques distincts :
- Une huile essentielle « volatile » représentant 50-90% de l'oléorésine, qui est majoritairement composée de sesquiterpènes. Parmi ces sesquiterpènes, on distingue majoritairement du germacrène D, (E)-β-caryophyllène, β- et δ-élémène, α-ylangenea, α -gurjunène, α-humulène. Les sesquiterpènes minoritaires sont les suivants α-cubébène, α-copaène, 7-epi-sesquithujène, cis- et trans- α-bergamotène, sesquisabinène-A et B, 4αH,10αH-guaia-1(5),6-diène, allo-aromadendrène, γ-uurolène, α-amorphène, β-selinène, bicycliosesquiphyllandrène, α-muurolène, β-bisabolène, γ-cadinène, δ-cadinène, cis-calamène, zonarène, cakina-1'4-diène, α-cadinène, α-calacorène, selina-3,7(11)-diène, germacrène B. Cette huile volatile est très limpide, incolore, d'une odeur et d'une saveur très prononcées.
- Une fraction « non volatile » représentant 10-50% de l'oléorésine, qui est composée majoritairement des acides diterpéniques et/ou esters d'acides diterpéniques suivants : l'acide copalique, l'acide copaiférolique, l'ester diméthylique de l'acide agathendioique, l'acide agathique, l'ester méthylique de l'acide 3beta-hydroxyanticopalique, l'acide hardwickiique, l'acide 7 alpha acetoxyhardwickiique. Ce résidu de distillation est un liquide visqueux, d'odeur aromatique, de couleur marron foncé.

KR10-0863616 décrit des utilisations cosmétiques et thérapeutiques diverses de la fraction volatile de l'oléorésine de Copaifera, c'est-à-dire l'huile essentielle. Ce document enseigne que cette huile essentielle disposerait de nombreuses vertus anti-rides, anti-inflammatoires, stimulation de la pousse des cheveux, anti-obésité, anti-oxydantes, immunosuppressives et blanchissantes pour la peau. En ce qui concerne les propriétés anti-alopécie alléguées dans ce document en ce qui concerne cette huile essentielle, celle-ci est appliquée topiquement sur le crane de patients chauves et l'effet sur la régénération de follicules pileux est constatée équivalent à celui observé avec du minoxidil. Cependant aucun effet sur la prévention de la chute n'est démontré.

Gomes da Silva et al (Alternative Medicine Review ; 17 ; 1 ; p 69-75) décrivent l'effet anti-acné de l'huile essentielle de Copaifera. Dans son introduction, cet article confirme que l'oléorésine connue pour nombre de propriétés anti-inflammatoires, anti-septiques et cicatrisantes est composée de deux fractions, une sesquiterpénique (comprise dans l'huile essentielle volatile) et une diterpénique (comprise dans la fraction non volatile). Dans les tests mis en oeuvre pas Gomes da Silva et al, l'huile essentielle (ie fraction volatile riche en sesquiterpènes) est appliquée sur des volontaires atteints d'acné de type 1 (comédons non enflammés). La conclusion de ces tests est que l'huile essentielle comprenant la fraction volatile sesquiterpénique de l'oléorésine de Copaiba pourrait avoir une utilité dans le traitement de l'acné légère et les auteurs évoquent un rôle potentiel inhibiteur de la croissance de Propionibacterium acnes dans l'effet anti-acné de cette huile essentielle.

La demande CN106038394 décrit une composition associant 5 extraits végétaux, dont l'huile essentielle de Copaifera. L'application de cette composition concerne l'acné et la perte en eau de la peau et nullement l'alopécie.

Le document internet « Prima fleur recommends treating skin to nourishing vegetable oil and essential oil serums to target any skin condition » (Massage magazine) décrit une association de 3 huiles : Copaifera, Euterpe et Carapa sans précision sur les ratios ni leur composition qualitative.

La demande WO 2013/084163 décrit une association de 2 actifs, à savoir le Chelidonium majus et la résine de Copaifera, pour son utilisation dans le traitement de l'hyper-prolifération épidermique et l'acné.

Veiga et al. (Veiga et al. Phytochemical and antioedematogenic studies of commercial copaiba oils available in Brazil. Phytother Res. 2001 15(6):476-80) décrit la composition d'huiles commerciales de Copaiba, sans distinguer entre les fractions volatiles et non volatiles.

La demande FR 2 816 843 concerne des inhibiteurs de l'enzyme 5-alpha-réductase élaborés à partir du resvératrol et de dérivés du resvératrol.

Leandro et al. (Leandro LM, Vargas Fde S, Barbosa PC, Neves JK, da Silva JA, da Veiga-Junior VF. Chemistry and biological activities of terpenoids from copaiba (Copaifera spp.) oleoresins. Molecules. 2012 17(4):3866-89) divulgue que Copaifera comprend dans son extrait de diterpènes l'acide ent-agathique.

La demande KR 2000 0042508 divulgue que l'acide agathique est un inhibiteur de la testostérone 5-alpha réductase.

De nombreuses études se sont donc focalisées sur la fraction volatile de l'oléorésine de Copaifera quant aux propriétés thérapeutiques de cette oléorésine.

### RESUME DE LA DIVULGATION

L'invention est définie dans les revendications annexées et tous les autres aspects ou modes de réalisation décrits ici sont fournis à titre informatif uniquement. En particulier, les références aux méthodes de traitement par thérapie ou aux méthodes de diagnostic in vivo dans cette description doivent être interprétées comme des références aux extraits de Copaifera et aux compositions dermatologiques de la présente invention destinés à être utilisés dans ces méthodes.

Plus particulièrement, il a été mis en évidence de façon tout à fait surprenante que l'oléorésine de Copaifera, via sa fraction non volatile, présente des propriétés dermatologiques et cosmétique particulièrement intéressantes et avantageuses et ce grâce à son effet inhibiteur de l'enzyme 5α-réductase.

Cette propriété est pertinente pour des utilisations en cosmétique et dermatologie dans le traitement ou la prévention de l'alopécie ainsi que dans le traitement de la séborrhée.

L'invention décrite ici a donc pour objet, dans un premier aspect, un extrait de Copaifera comprenant, à titre de principe actif inhibiteur de la 5-alpha réductase, un mélange d'acides diterpéniques et/ou esters d'acides diterpéniques, pour son utilisation dans la prévention et/ou le traitement de l'alopécie, caractérisé en ce que l'extrait consiste en la fraction non volatile de l'oléorésine de Copaifera et que le mélange d'acides diterpéniques et/ou esters d'acides diterpéniques comprend au moins 6, voire au moins 7 acides diterpéniques et/ou esters d'acides diterpéniques choisis dans le groupe constitué par : l'acide copalique, l'acide copaiférolique, l'ester diméthylique de l'acide agathendioique, l'acide agathique, l'ester méthylique de l'acide 3beta-hydroxyanticopalique, l'acide hardwickiique, l'acide 7 alpha acetoxyhardwickiique.

Dans un mode de réalisation particulier, le Copaifera est choisi parmi les espèces végétales suivantes : Copaifera officinalis, Copaifera multijugla et Copaifera reticulata ; utilisés seules ou en mélange.

Dans un mode de réalisation particulier, l'extrait contient, en poids par rapport au poids total de l'extrait, entre 7,5 et 95% d'acides diterpéniques et/ou esters d'acides diterpéniques.

Dans un mode de réalisation particulier, le mélange d'acides diterpéniques et/ou esters d'acides diterpéniques comprend les acides diterpéniques et/ou esters d'acides diterpéniques suivants : l'acide copalique, l'acide copaiférolique, l'ester diméthylique de l'acide agathendioique, l'acide agathique, l'ester méthylique de l'acide 3beta-hydroxyanticopalique, l'acide hardwickiique, l'acide 7 alpha acetoxyhardwickiique.

Dans un deuxième aspect, l'invention vise une composition dermatologique comprenant un extrait de Copaifera tel que décrit ci-dessus, avec au moins un excipient dermatologiquement acceptable, pour son utilisation dans le traitement de l'alopécie.

Dans un mode de réalisation particulier, l'extrait de Copaifera est le seul principe actif anti-alopécie de ladite composition.

Dans un mode de réalisation particulier, la quantité d'extrait de Copaifera est comprise entre 0,05% à 10% en poids par rapport au poids total de la composition.

Dans un mode de réalisation particulier, la composition décrite ci-dessus est utilisée pour ralentir la chute des cheveux.

Dans un mode de réalisation particulier, la forme de la composition décrite ci-dessus est adaptée à une administration topique.

### DESCRIPTION DETAILLEE

L'alopécie se définit comme une perte de cheveux ou de poils, partielle ou totale. La vie d'un cheveu est soumise à un cycle dit cycle pilaire au cours duquel trois phases se succèdent. La phase anagène, c'est une période de croissance active et continue, associée avec une intense activité métabolique au niveau du bulbe. La phase catagène au cours de laquelle, les activités mitotiques sont ralenties. Le cheveu subit une involution, le follicule s'atrophie et son implantation dermique apparaît de plus en plus haute. La dernière phase est la phase télogène qui correspond à une période de repos du follicule et le cheveu fini par tomber poussé par un nouveau cheveu. Le cycle pilaire est achevé, un autre peut débuter. Il y a environ 20 à 25 cycles par vie de bulbes chez l'homme. Au cours du vieillissement les cheveux deviennent plus fins et leurs cycles plus courts.

L'alopécie dite androgénétique est due à une accélération du cycle pilaire (raccourcissement de la durée du cycle pilaire), phénomène qui conduit dans un premier temps à l'apparition de cheveux miniaturisés dits « vellus » puis à un épuisement prématuré du renouvellement des cheveux. En effet, la durée de la phase anagène (phase de croissance) se raccourcie et passe de plusieurs années (2 à 5 ans) à quelques mois voire quelques semaines (Whiting et al, J Investig Dermatol Symp Proc, 1999). La conséquence est une chute précoce des cheveux.

Il est connu à ce jour que les mécanismes responsables de l'alopécie androgénétique héréditaire (que l'on appelait avant alopécie séborrhéique) mettent entre autres en jeu la composante hormonale avec la surexpression du récepteur aux androgènes (= récepteur de la Testostérone et de la DHT) et une activité plus intense de l'enzyme 5 alpha-réductase. Cette dérégulation hormonale entraîne une production excessive de dihydrotestostérone, métabolite actif de la testostérone. Au niveau de la papille dermique, ce métabolite va stimuler la production d'inhibiteurs du cycle pilaire entraînant un raccourcissement de la phase anagène forçant le cheveu à passer trop vite en phase télogène et ne laissant pas au follicule pileux assez de temps pour fabriquer une kératine de qualité et nécessairement après plusieurs cycles, un épuisement de la capacité du follicule pileux à produire une tige pilaire.

Cette alopécie due à un excès d'androgènes touche également les femmes au moment de la ménopause (alopécie post-ménopausique) ou à la suite d'un traitement par des androgènes. Elle commence au niveau des tempes et à la couronne. Cette chute de cheveux est plus diffuse et étendue que chez l'homme. La perte de cheveux concerne la totalité du cuir chevelu, de manière homogène.

Un actif inhibiteur de 5 alpha réductase permet ainsi de traiter et/ou prévenir la chute de cheveux chez l'homme et/ou la femme.

Quant à la séborrhée, elle correspond à une production excessive de sébum par les glandes sébacées. Globalement, l'homme possède 2 000 000 de glandes sébacées annexées à 6 000 000 de poils et cheveux. La répartition des glandes sébacées est non uniforme. La densité des glandes sébacées atteint 300 à 900 glandes sébacées/cm 2 au niveau du visage et du cuir chevelu, et cette densité est de l'ordre de 100 glandes sébacées/cm 2 dans la partie haute du thorax et du dos.

L'activité de la glande sébacée est influencée par les androgènes. Les androgènes ne sont actifs que sous l'influence de l'enzyme 5-alpha réductase qui assure la métabolisation des androgènes au niveau la glande sébacée ce qui induit la production de sébum. Une hyperactivation de l'enzyme 5-□ réductase provoque la séborrhée.

Le siège des manifestations de la séborrhée est la région médio-faciale (front, nez menton) où les glandes sébacées sont les plus nombreuses et les plus volumineuses. La séborrhée se manifeste également au niveau du cuir chevelu où elle prédomine dans les régions frontale, fronto-temporale et au sommet du crâne.

La séborrhée occasionne des désagréments d'ordre esthétique et dermatologiques tels que la dermite séborrhéique. En ce qui concerne la peau, elle présente un aspect luisant, le teint est terne et les orifices pilo-sébacés sont dilatés. De plus, le maquillage ne présente pas une bonne tenue sur ce type de peaux dites grasses.

Pour la séborrhée du cuir chevelu, les cheveux présentent un aspect gras et terne et ils sont difficiles à coiffer. Quand la séborrhée est intense, elle est dite huileuse, fluente et elle peut être associée à une odeur rance.

La séborrhée est souvent associée à l'alopécie androgénétique.

Un actif inhibiteur de l'activité de l'enzyme 5-□ réductase permet donc de réduire la sécrétion de sébum, traiter la séborrhée et résoudre les désagréments d'ordre esthétique liés à la séborrhée.

Ainsi, la présente divulgation concerne, dans un premier mode de réalisation, un extrait de Copaifera comprenant ou consistant en, à titre de principe actif inhibiteur de la 5-alpha réductase, un mélange d'acides diterpéniques et/ou esters d'acides diterpéniques, pour son utilisation dans la prévention et/ou le traitement les troubles dermatologiques choisis parmi la séborrhée et l'alopécie. De préférence, il s'agit du traitement de l'alopécie.

Dans un second mode de réalisation de l'divulgation, le Copaifera est choisi parmi les espèces végétales suivantes : *Copaifera officinalis, Copaifera multijugla* et *Copaifera reticulata ;* utilisées seules ou en mélange.

L'extrait de Copaifera comprenant un mélange d'acides diterpéniques et/ou esters d'acides diterpéniques divulgué ici peut être un extrait végétal de Copaifera raffiné et contenant, en poids par rapport au poids total de l'extrait, entre 7,5 et 95% d'acides diterpéniques et/ou esters d'acides diterpéniques.

De manière avantageuse l'extrait de Copaifera divulgué ici comprend, ou consiste en, une oléorésine de Copaifera contenant au moins 7,5 % en poids d'un mélange d'acides diterpéniques et/ou esters d'acides diterpéniques, particulièrement au moins 15%, plus particulièrement au moins 20%, plus particulièrement encore au moins 25% ; par rapport au poids total de l'oléorésine de Copaifera.

L'extrait de Copaifera divulgué ici peut comprendre, ou consister en, une oléorésine de Copaifera enrichie en un mélange d'acides diterpéniques et/ou ester d'acides diterpéniques contenant, en % en poids, entre 48 et 90% d'acides diterpéniques et/ou esters d'acides diterpéniques, plus particulièrement entre 50 et 90%, plus particulièrement entre 60 et 90%, plus particulièrement entre 70 et 90%, plus particulièrement entre 80 et 90% ; par rapport au poids total de l'oléorésine de Copaifera enrichie.

La divulgation vise aussi, un extrait de Copaifera divulgué ici pour son utilisation en tant que principe actif anti-séborrhée dans une composition dermatologique pour traiter et/ou prévenir la séborrhée, caractérisé en ce que ledit extrait est le seul principe actif anti-séborrhée de ladite composition.

La divulgation vise aussi un extrait de Copaifera divulgué ici pour son utilisation en tant que principe actif anti-alopécie dans une composition dermatologique pour traiter et/ou prévenir l'alopécie, caractérisé en ce que ledit extrait est le seul principe actif anti-alopécie de ladite composition.

Par l'expression « enrichie », on entend ici le fait que l'oléorésine de l'extrait, présente une concentration d'acides diterpéniques et/ou esters d'acides diterpéniques accrue par rapport à l'oléorésine native - ie obtenue directement de l'arbre - et ce par divers procédés d'augmentation de cette concentration en lesdits acides diterpéniques et/ou ester d'acides diterpéniques ; cette augmentation de la concentration se faisant préférentiellement pour lesdits acides et esters comparativement aux autres composés de l'oléroésine, qui eux ne sont pas ou que peu concentrés. Un enrichissement peut se réaliser par un traitement via un procédé visant à concentrer la teneur en ces 7 acides diterpéniques et/ou esters d'acides diterpéniques par rapport aux autres composés et molécules. Un tel enrichissement comprend principalement l'élimination au moins partielle de la fraction volatile (ie huile essentielle).

Le mélange d'acides diterpéniques et/ou d'esters d'acides diterpéniques comprend au moins 2, voire au moins 3, voire au moins 4, voire au moins 5, voire au moins 6, voire au moins 7 acides diterpéniques et/ou esters d'acides diterpéniques choisis dans le groupe consitué par : l'acide copalique, l'acide copaiférolique, l'ester diméthylique de l'acide agathendioique, l'acide agathique, l'ester méthylique de l'acide 3beta-hydroxyanticopalique, l'acide hardwickiique, l'acide 7 alpha acetoxyhardwickiique . De préférence il s'agit des 7 acides diterpéniques et/ou esters d'acides diterpéniques cités.

**Tableau 1 : Formules brutes et développées des différents acides diterpéniques et/ou esters d'acides diterpéniques**

| Nom | Formule brute | m/z | Formule développée |
|---|---|---|---|
| Acide copalique | C20H32O2 | 304 | 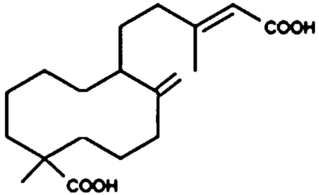 |
| Acide copaiférolique | C20H32O3 | 320 | 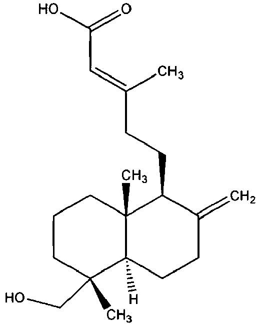 |
| Ester diméthylique de l'acide agathendioique | C22H34O4 | 362 | 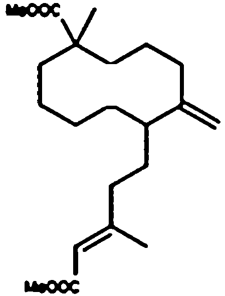 |
| Acide agathique | C20H30O4 | 334 | 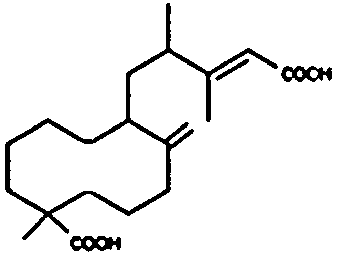 |
| Ester méthylique de l'acide 3beta-hydroxyanticop alique | C20H30O4 | 334 | 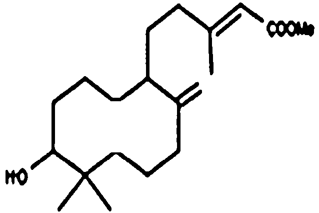 |
| | | | |
| Acide hardwickiique | C20H28O3 | 316 | 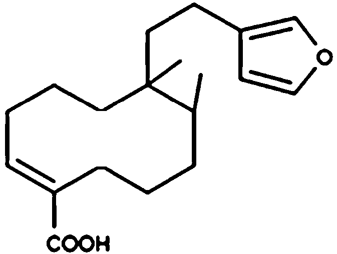 |
| Acide 7-alpha acetoxyhardwic kiique | C22H30O5 | 374 | 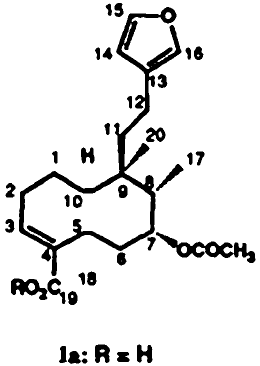 |

De préférence le mélange d'acides diterpéniques et/ou d'esters d'acides diterpéniques comprend tous les acides diterpéniques et/ou esters d'acides diterpéniquessuivants : l'acide copalique, l'acide copaiférolique, l'ester diméthylique de l'acide agathendioique, l'acide agathique, l'ester méthylique de l'acide 3beta-hydroxyanticopalique, l'acide hardwickiique, l'acide 7 alpha acetoxyhardwickiique.

L'extrait de Copaifera divulgué ici consistant en une oléorésine comprend, en pourcentage en poids par rapport au poids total de l'extrait, entre 7,5 et 40 % des 7 acides diterpéniques et/ou ester d'acides diterpéniques mentionnés ci-avant, préférentiellement entre 10 et 30%.

L'extrait de Copaiefera divulgué ici consistant en une oléorésine enrichie comprend, en pourcentage en poids par rapport au poids total de l'extrait, entre 48 et 90 % des 7 acides diterpéniques et/ou ester d'acides diterpéniques mentionnés ci-avant, préférentiellement entre 60 et 85%.

**Tableau 2**

| Dénomination | Teneur massique dans l'oléorésine (%) | Teneur massique en fraction diterpénique (%) |
|---|---|---|
| Acide copalique | 3 à 13 | 15 à 50 |
| Acide hardwickiique | 0.5 à 1.5 | 2 à 15 |
| Acide copaiférolique | 1 à 6 | 10 à 30 |
| Acide agathique et Ester méthylique de l'acide 3beta-hydroxyanticopalique | 1 à 6 | 8 à 30 |
| Ester diméthylique de l'acide agathendioique | 1 à 6 | 10 à 30 |
| Acide 7-alpha acetoxyhardwickiique | 1 à 8 | 3 à 15 |

Dans un mode de réalisation particulier, l'extrait de Copaifera divulgué ici comprend ou consiste en la fraction non volatile de l'oléorésine de Copaifera.

Dans un autre mode de réalisation, l'extrait de Copaifera divulgué ici comprend ou consiste en un mélange d'acides diterpéniques et/ou esters d'acides diterpéniques .

Cette fraction « non volatile » de l'oléorésine de Copaifera comprend le mélange d'acides diterpéniques et/ou esters d'acides diterpéniques et peut être obtenue après élimination totale ou partielle, de préférence élimination totale, de l'huile essentielle, notamment par hydrodistillation. Cette fraction non volatile comprend au moins 80 % et de préférence entre 80 et 90% en poids d'acides diterpéniques et/ou esters d'acides diterpéniques par rapport au poids total de ladite fraction. Cela représente un extrait consistant en oléorésine enrichie dont l'enrichissement est maximal. ;.

Le mélange d'acides diterpéniques et/ou esters d'acides diterpéniques peut aussi être obtenu à partir d'une oléorésine de Copaifera, ou de la fraction « non volatile » de l'oléorésine de Copaifera ; notamment par extraction liquide-liquide jusqu'à obtenir un mélange présentant une concentration en acides diterpéniques et/ou esters d'acides diterpéniques comprise entre 50 et 100%, particulièrement entre 60 et 100%, plus particulièrement encore entre 80 et 100% en poids d'acides diterpéniques et/ou esters d'acides diterpéniques par rapport au poids total de la fraction liquide obtenue après extraction et l'élimination du solvant d'extraction. Cette fraction liquide obtenue après extraction et élimination du solvant représente ledit mélange.

La présente divulgation vise encore une composition dermatologique ou cosmétique comprenant un extrait de Copaifera selon la divulgation et tel que décrit supra, avec au moins un excipient dermatologiquement ou cosmétiquement acceptable, pour son utilisation dans le traitement de l'alopécie et/ou de la séborrhée.

Selon un mode de réalisation, la divulgation vise une composition dermatologique ou cosmétique comprenant un extrait de Copaifera tel que décrit supra, avec au moins un excipient dermatologiquement ou cosmétiquement acceptable, pour son utilisation dans le traitement de l'alopécie, caractérisé en ce que l'extrait de Copaifera est le seul principe actif anti-alopécie de ladite composition.

De manière préférée, le traitement de l'alopécie vise à ralentir la chute des cheveux.

Selon un mode de réalisation, la divulgation vise une composition dermatologique ou cosmétique comprenant un extrait de Copaifera divulgué ici et tel que décrit supra, avec au moins un excipient dermatologiquement ou cosmétiquement acceptable, pour son utilisation dans le traitement de la séborrhée, caractérisé en ce que l'extrait de Copaifera est le seul principe actif anti-séborrhée de ladite composition. De manière particulière, la séborrhée concerne la séborrhée du cuir chevelu et/ou de la peau, et de préférence la séborrhée du cuir chevelu.

De manière préférée la quantité d'extrait de Copaifera est comprise entre 0,05% à 10% en poids par rapport au poids total de la composition.

La divulgation vise encore une composition telle que divulguée ici pour son utilisation dans le traitement de l'alopécie et/ou de la séborrhée, sous une forme adaptée à une administration topique. De manière avantageuse l'extrait de Copaifera est le seul actif anti-alopécie de la composition pour son utilisation dans le traitement de l'alopécie. Selon un autre mode avantageux, l'extrait de Copaifera est le seul actif anti séborrhée de la composition pour utilisation dans le traitement de la séborrhée.

Le traitement ou la prévention de l'alopécie peut être le ralentissement de la chute des cheveux.

La séborrhée peut être choisie parmi la séborrhée de la peau et la séborrhée du cuir chevelu.

L'administration topique peut être une administration sur les cheveux et/ou le cuir chevelu et/ou la peau.

La divulgation vise encore une composition telle que divulguée ici pour son utilisation dans le traitement de l'alopécie et/ou de la séborrhée, sous une forme adaptée à une administration orale. Une composition selon la divulgation est caractérisée en ce qu'elle comprend de 0,05% à 10% en poids d'extrait selon la divulgation par rapport au poids total de la composition. Dans un mode particulier, l'extrait de Copaifera divulgué ici est le seul actif pour le traitement de l'alopécie de ladite composition. Selon un autre mode particulier, l'extrait de Copaifera divulgué ici est le seul actif pour le traitement de la séborrhée de la composition.

La divulgation vise encore l'utilisation d'un extrait de Copaifera divulgué ici pour le traitement et/ou la prévention de la séborrhée et/ou de l'alopécie.

La divulgation vise encore une composition anti-seborrhéique comprenant un extrait de Copaifera décrit plus avant, avec au moins un excipient dermatologiquement ou cosmétiquement acceptable. La composition anti-seborrhéique préférée est une composition adaptée pour une administration topique. De manière avantageuse, la composition anti-séborrhéique selon la divulgation ne contient que l'extrait de Copaifera divulgué ici comme actif anti-séborrhéique.

La divulgation vise encore une composition anti-alopécie comprenant un extrait de Copaifera décrit plus avant, avec au moins un excipient dermatologiquement ou cosmétiquement acceptable. La composition anti-alopécie préférée est une composition adaptée pour une administration topique. De manière avantageuse, la composition anti-alopécie selon la divulgation ne contient que l'extrait de Copaifera divulgué ici comme actif anti-alopécie.

La composition selon la divulgation telle que décrite plus avant est caractérisée en ce que la quantité d'extrait de Copaifera est compris entre 0,05% à 10% en poids par rapport au poids total de la composition. La quantité d'extrait pourra être adaptée en fonction de la nature de l'extrait, i.e. selon qu'il s'agit d'oléorésine brute, d'oléorésine enrichie ou du mélange d'acides diterpéniques et/ou esters d'acides diterpéniques . Cette quantité pourra être comprise entre 0.05 et 10% en poids par rapport au poids total de la composition, plus particulièrement entre 1 et 10%, entre 2 et 7.5% ou encore entre 3 et 6% par exemple.

Dans un mode de réalisation particulier, l'extrait de Copaifera dans la composition selon la divulgation comprend ou consiste en la fraction non volatile de l'oléorésine de Copaifera.

Dans un autre mode de réalisation, la présente divulgation vise aussi une méthode cosmétique, en particulier une méthode non thérapeutique, de traitement ou de prévention d'un désordre choisi parmi la peau grasse, la peau luisante, les cheveux gras, le cuir chevelu gras, et de préférence les cheveux gras, le cuir chevelu gras ; comprenant l'application topique d'un extrait selon la divulgation ou d'une composition divulguée ici. Selon un mode de réalisation particulier, la méthode cosmétique, en particulier non thérapeutique, est caractérisée en ce que l'extrait de Copaifera divulgué ici est le seul actif destiné au traitement ou à la prévention d'un désordre choisi parmi la peau grasse, la peau luisante, les cheveux gras, le cuir chevelu gras et de préférence les cheveux gras, le cuir chevelu gras.

Dans un autre mode de réalisation, la présente divulgation vise aussi une méthode cosmétique, en particulier non thérapeutique, de matification de la peau séborrhéique.

L'extrait selon la divulgation peut être associé ou mélangé à un support ou véhicule de nature lipidique afin de standardiser la teneur en acides diterpéniques et/ou esters d'acides diterpéniques dans la composition divulguée ici.

Le support ou véhicule lipidique peut être une huile, en particulier une huile dermatologiquement ou cosmétiquement acceptable.

Dans la présente divulgation, on entend désigner par « dermatologiquement ou cosmétiquement acceptable » ce qui est utile dans la préparation d'une composition dermatologique ou cosmétique, qui est généralement sûr, non toxique et ni biologiquement ni autrement non souhaitable et qui est acceptable pour une utilisation dermatologique ou cosmétique notamment par application topique.

Les compositions décrites ici sont avantageusement destinées à une application topique, en particulier sur la peau.

Les compositions divulguées ici pourront ainsi se présenter sous les formes qui sont habituellement connues pour une administration topique, c'est à dire notamment les lotions, les mousses, les gels, les dispersions, les émulsions, les sprays, les sérums, les masques ou les crèmes, avec des excipients permettant notamment une pénétration cutanée afin d'améliorer les propriétés et l'accessibilité du principe actif.

Avantageusement, il s'agira d'une crème.

Ces compositions contiennent généralement, outre l'extrait selon la présente divulgation, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales, etc.

Ces compositions peuvent en outre contenir d'autres principes actifs conduisant à un effet complémentaire ou éventuellement synergique.

### EVALUATION PHARMACOLOGIQUE

L'exemple suivant illustre la divulgation sans en limiter la portée.

Exemple 1 : Effets de différents composés sur l'activité de 5α-réductase de fibroblastes issus de follicules humains de papille dermique.

L'objectif de cette étude était d'évaluer une activité inhibitrice éventuelle de différents composés sur la 5α-réductase.

### Matériel et méthodes

L'étude est réalisée sur des cellules humaines issues de follicule de papilles dermiques de donneurs. Ce modèle est intéressant, en effet les papilles dermiques présentent l'isoforme 5alpha2 comme dans le tissu prostatique. Les cellules sont ensemencées dans des plaques de 24 puits et mises en culture pendant 24h dans un milieu de culture DMEM supplémenté en L-glutamine (2mM), de pénicilline (50U/ml), de streptomycine (50µg/ml) et de Fetal Calf Serum (10%) dans des conditions de cultures standards (37°C et 5% CO₂). Le milieu de culture est ensuite remplacé par un milieu d'analyse DMEM supplémenté en L-glutamine (2mM), de pénicilline (50U/ml), de streptomycine (50µg/ml) et de Fetal Calf Serum (1%). Ce milieu d'analyse peut contenir ou pas (conditions contrôles) les produits à tester et un composé servant de référence le finastéride (10µM) pendant 24h de pré-incubation. Les cellules étaient ensuite traitées avec un milieu d'analyse contenant de la testostérone [C¹⁴] et contenant ou pas (conditions contrôles) les produits à tester ou de référence, et les cellules étaient incubées pendant 24h dans ces conditions. Après incubation, les surnageants étaient collectés pour l'analyse du métabolisme de la testostérone. Toutes les expériences ont été réalisées 3 fois. Les molécules stéroïdes étaient extraites des surnageants avec un mélange chloroforme/méthanol. La phase organique était collectée et les différentes espèces moléculaires (métabolites de la testostérone) étaient séparées par chromatographie sur couche mince en utilisant un système de solvants contenant du dichlorométhane, de l'éthylacétate et du méthanol. Une autoradiographie était réalisée sur la chromatographie et la testostérone transformée était estimée par analyse densitométrique.

Ainsi la métabolisation de la testostérone en dihydrotestostérone rend compte de l'activité 5α-réductase, elle est évaluée par le ratio dihydrotestostérone/testostérone.

### Résultats

Un premier groupe d'expériences permet de mettre en évidence les effets de l' oléorésine de *Copaifera officinalis* (tableau 3 ci-dessous). De façon surprenante, les inventeurs ont mis en évidence une activité significative et reproductible de l'inhibition de la 5α-réductase par l'oléorésine de Copaifera officinalis, cette inhibition apparaît même concentration-dépendante. L'inhibition importante de cette enzyme par le finastéride permet de bien valider l'ensemble de ces expériences.

**Tableau 3 : Effets de l'oléorésine de Copaifera officinalis et du finastéride sur la métabolisation de la testostérone / la production de dihydrotestostérone (activité 5α-réductase ; n=3)**

| Contrôle | Finastéride | Oléorésine (C. officinalis) | |
|---|---|---|---|
| | 10 µM | 10 µg/ml | 30 µg/ml |
| 100 | -77% | -15% | -27% |
| | ** P<0,01 | * P<0,05 | ** P<0,01 |

L'étude statistique est réalisée versus le groupe contrôle (test de Dunnett).

L'oléorésine testée a été préparée selon la méthode décrite dans l'exemple a.

A titre de comparaison, un extrait de *Serenoa repens,* un extrait de *Curcubita pepo* ainsi que le composé glycéryl laurate ont également été testés dans une de ces expériences.

A 10 µg/ml l'extrait de Serenoa repens n'induit pas d'inhibition significative de la 5α-réductase. En revanche à 20 µg/ml cet extrait induit une inhibition de 23% atteignant la significativité statistique (p<0,05 versus contrôle).

Une inhibition significative de 17% est obtenue pour un extrait de *Curcubita pepo* testé à 100µg/ml.

Une inhibition significative de 19% est obtenue pour le glycéryl laurate testé à 40µg/ml.

Une seconde série d'expériences a été menée pour évaluer si l'activité inhibitrice sur 5α-réductase était portée par la fraction non volatile ou plutôt par la fraction volatile correspondant à l'huile essentielle. Les résultats sont résumés dans le tableau 4 ci-dessous. La préparation des fractions volatile et non volatile est réalisée selon la méthode décrite dans l'exemple c avec le diéthylester comme solvant apolaire..

**Tableau 4 : Effets des fractions non volatile et volatile issues de l'oléorésine de Copaifera officinalis sur la métabolisation de la testostérone / la production de dihydrotestostérone (activité 5α-réductase)**

| Contrôle | Fraction non volatile | | | Fraction volatile | |
|---|---|---|---|---|---|
| | 0,3 µg/ml | 1 µg/ml | 3 µg/ml | 7,7 µg/ml | 23,1 µg/ml |
| 100 | 0 | -6% | -14% | +2 | +9 |
| | P=NS | P=NS | * P<0,05 | P=NS | P=NS |

L'étude statistique est réalisée versus le groupe contrôle (test de Dunnett).

Il s'avère que l'activité de l'oléorésine de Copaifera officinalis est portée par la fraction non volatile, en effet aucune activité de la fraction volatile n'a été mise en évidence. L'inhibition de la 5α-réductase n'est que de 14% à 3 µg/mL (10 x moins concentré que l'oléorésine) mais cette réduction atteint la significativité statistique (p<0,05).

Une troisième série d'expériences a été mise en place afin de montrer que d'autres espèces de Copaifera, notamment C. multijuga présentaient également une activité intéressante sur l'inhibition de la 5α-réductase. Les inventeurs se sont focalisés sur la fraction non volatile, fraction portant l'activité inhibitrice. Les résultats sont résumés dans le tableau 3 ci-dessous.

**Tableau 5 : Effets des fractions diterpénique issues de la résine de Copaifera multijuga sur la métabolisation de la testostérone / la production de dihydrotestostérone (activité 5α-réductase ; n=2)**

| Contrôle | Fraction non volatile | |
|---|---|---|
| | Copaifera multijuga | |
| | 1 µg/ml | 10 µg/ml |
| 100 | 0 | -31% |
| | P=NS | ** P<0,01 |

L'étude statistique est réalisée versus le groupe contrôle (test de Dunnett).

La préparation des fractions volatile et non volatile est réalisée selon la méthode décrite dans l'exemple c avec le diéthylester comme solvant apolaire..

Ces résultats montrent clairement que plusieurs espèces de Copaifera présentent un intérêt. En effet, la fraction non volatile de l'espèce C. multijuga atteint 31 % d'inhibition de la 5α-réductase à 10µg/ml.

L'ensemble de ces résultats permet de conclure que l'oléorésine de Copaifera présente une activité inhibitrice sur la 5α-réductase extrêmement intéressante. Cette activité est portée par la fraction non volatile de la résine. Enfin les inventeurs ont également montré que cette activité est retrouvée sur plusieurs espèces de Copaifera.

### EXEMPLES

### - Préparation de l'oléorésine

### Exemple a :

L'écorce de tronc des arbres de *Copaifera officinalis* et/ou *Copaifera multijuga et*/*ou Copaifera reticulata* est entaillée afin de récupérer l'oléorésine. Celle-ci est ensuite homogénéisée puis stabilisée sous azote.

La matière active est composée à 100% d'oléorésine brute de tronc de Copaifera officinalis et/ou *Copaifera officinalis* et/ou *Copaifera. multijuga et*/*ou Copaifera. Reticulata.*

### Analyse LCMS d'une oléorésine de Copaifera Officinalis:

Chaque échantillon a été analysé par UHPLC-QTOFMS selon un gradient linéaire classique.

*Séparation sur système Waters Acquity UHPLC.*
- Colonne 100 x 2.1 mm, 1.7 µm, Acquity BEH C18 équipé d'une pré-colonne
- Phase mobile :
Phase mobile A : Eau LCMS grade + 0.1 % acide formique
Phase mobile B : Acétonitrile LCMS grade + 0.1 % acide formique

- Gradient :

| Temps (min) | %A | %B |
|---|---|---|
| 0-0.5 | 50 | 50 |
| 0.5-4 | 50→ 40 | 50 → 60 |
| 4-12 | 40→ 1 | 60 →99 |
| 12-15 | 1 | 99 |
| 15-15.5 | 1 →50 | 99→50 |
| 15.5-19 | 50 | 50 |

*Acquisitions :* UV 220 nm

| Structure | m/z | Teneur dans l'oléorésine |
|---|---|---|
| | | (% massique par rapport au poids de l'oléorésine) |
| Acide Copalique | 304 | 5,69 |
| Acide Hardwickiique | 316 | 0,61 |
| Acide Copaiferolique | 320 | 2,90 |
| Acide Agathique | 334 | 2,35 |
| Et ester méthylique de l'acide 3 beta-hydroxyanticopalique | | |
| Ester diméthylique de l'acide agathendioique | 362 | 2,92 |
| Acide 7α-acetoxyhardwickiique | 374 | 0,79 |

- Préparation de fraction non volatile.
- Exemple b :
L'oléorésine obtenue selon l'exemple précédent a) est mise en suspension dans 10 volumes d'eau chauffée à 100 °C pendant 4 h pour réaliser une hydrodistillation. L'huile essentielle, volatile, est récupérée par condensation Après l'hydrodistillation, le résidu de distillation est récupéré. Après séchage par lyophilisation ou autre moyen de séchage, le résidu constitue la fraction non volatile.
- Exemple c :
1 volume d'oléorésine de Copaifera obtenue selon l'exemple précédent a) est diluée dans 8 à 10 volumes d'un solvant lipophile non miscible à l'eau (tel que le diéthyl éther, l'acetate d'éthyle). Cette solution est extraite par extraction liquide/liquide avec une solution de soude (NaOH) à 5 %. L'opération est répétée 3 fois avec 4 à 5 V de NaOH 5 %. La phase inférieure (phase aqueuse basique) est acidifiée par addition d'acide chlorhydrique (HCl) 1 N puis extraite par extraction liquide/liquide avec un solvant apolaire non miscible avec l'eau (comme de dithyléther, l'acétate d'ethyle). La phase acétate d'éthyle est lavée à l'eau puis déshydratée sur Na2SO4. Après élimination du solvant par rotavapor ou autres moyens de séchage, le résidu sec obtenu correspond au mélange d'acides diterpéniques et/ou esters d'acides diterpéniques selon la divulgation.

| Structure | m/z | Teneur massique dans la fraction non volatile |
|---|---|---|
| Acide Copalique | 304 | 28,45 |
| Acide Hardwickiique | 316 | 3,05 |
| Acide Copaiferolique | 320 | 14,52 |
| Acide Agathique | 334 | 11,77 |
| Et ester méthylique de l'acide 3 beta-hydroxyanticopalique | | |
| Ester diméthylique de l'acide agathendioique | 362 | 14,58 |
| Acide 7α-acetoxyhardwickiique | 374 | 3,96 |

- Exemple Composition capillaire anti-chute des cheveux (exemple illustratif)
Oléorésine de Copaifera Officinalis selon l'exemple a) de 0,05 à 10 %
DEXPANTHENOL de 0,3 à 1%
ALCOOL ISOPROPYLIQUE de 1 à 5%
PPG-26-BUT.-26/PEG-40 de 2 à 10%
ETHYLIQUE ALCOOL de 10 à 40%
PARFUM de 0,2 à 1%
EAU Qsp

- Exemple Composition anti-séborrhée (exemple illustratif)
Fraction non volatile selon l'exemple b : de 0,05 à 10 %
Glycérine : de 2% à 5%
Phénoxyéthanol : de 0,3 à 0,5%
Na2EDTA : de 0,1 à 0,2%
Polyacrylate-13 et Polyisobutène et Polysorbate 20 & eau : de 1% à 2%
Stéarate de glycéryle et PEG-100 Stéarate : de 2 à 5%
Cyclopentasiloxane : de 3% à 5%
Alcool cétylique : de 1% à 2%
Glycérol tri-2-éthylhexanoate : de 2% à 3%
Carbonate de dicapryle : de 2% à 3%
Polyméthylacrylate : de 2% à 3%
Parfum : de 0,1% à 1%
Eau Qsp

## Revendications

1. Extrait de Copaifera comprenant, à titre de principe actif inhibiteur de la 5-alpha réductase, un mélange d'acides diterpéniques et/ou esters d'acides diterpéniques, pour son utilisation dans la prévention et/ou le traitement de l'alopécie, **caractérisé en ce que** l'extrait consiste en la fraction non volatile de l'oléorésine de Copaifera et que le mélange d'acides diterpéniques et/ou esters d'acides diterpéniques comprend au moins 6, voire au moins 7 acides diterpéniques et/ou esters d'acides diterpéniques choisis dans le groupe constitué par : l'acide copalique, l'acide copaiférolique, l'ester diméthylique de l'acide agathendioique, l'acide agathique, l'ester méthylique de l'acide 3beta-hydroxyanticopalique, l'acide hardwickiique, l'acide 7 alpha acetoxyhardwickiique.

2. Extrait pour son utilisation selon la revendication 1, **caractérisé en ce que** le *Copaifera* est choisi parmi les espèces végétales suivantes : *Copaifera officinalis, Copaifera multijugla* et *Copaifera reticulata* ; utilisés seules ou en mélange.

3. Extrait pour son utilisation selon l'une des revendications 1 ou 2, **caractérisé en ce qu'**il contient, en poids par rapport au poids total de l'extrait, entre 7,5 et 95% d'acides diterpéniques et/ou esters d'acides diterpéniques.

4. Extrait pour son utilisation selon l'une des revendications 1 à 3, **caractérisé en ce que** le mélange d'acides diterpéniques et/ou esters d'acides diterpéniques comprend les acides diterpéniques et/ou esters d'acides diterpéniques suivants : l'acide copalique, l'acide copaiférolique, l'ester diméthylique de l'acide agathendioique, l'acide agathique, l'ester méthylique de l'acide 3beta-hydroxyanticopalique, l'acide hardwickiique, l'acide 7 alpha acetoxyhardwickiique.

5. Composition dermatologique comprenant un extrait de Copaifera selon l'une des revendications 1 à 4, avec au moins un excipient dermatologiquement acceptable, pour son utilisation dans le traitement de l'alopécie.

6. Composition pour son utilisation selon la revendication 5, **caractérisée en ce que** l'extrait de Copaifera est le seul principe actif anti-alopécie de ladite composition.

7. Composition pour son utilisation selon la revendication 5 à 6, **caractérisée en ce que** la quantité d'extrait de Copaifera est comprise entre 0,05% à 10% en poids par rapport au poids total de la composition.

8. Composition selon l'une des revendications 5 ou 6, pour son utilisation pour ralentir la chute des cheveux.

9. Composition pour son utilisation selon l'une des revendications 5 à 8, sous une forme adaptée à une administration topique.

## Patentansprüche

1. Copaifera-Extrakt, der als 5-alpha-Reduktasehemmenden Wirkstoff ein Gemisch aus Diterpensäuren und/oder Diterpensäureestern zu seiner Verwendung bei der Vorbeugung und/oder Behandlung der Alopezie umfasst, **dadurch gekennzeichnet, dass** der Extrakt aus der nichtflüchtigen Fraktion des Copaifera-Oleoresins besteht und dass das Gemisch aus Diterpensäuren und/oder Diterpensäureestern mindestens 6, sogar mindestens 7 Diterpensäuren und/oder Diterpensäureester umfasst, die aus der Gruppe ausgewählt sind, die besteht aus: Copalsäure, Copaiferolsäure, Dimethylester der Agathendiosäure, Agathsäure, dem Methylester der 3beta-Hydroxyanticopalsäure, Hardwickiinsäure, 7-alpha-Acetoxyhardwickiinsäure.

2. Extrakt zu seiner Verwendung nach Anspruch 1, **dadurch gekennzeichnet, dass** *Copaifera* aus den folgenden Pflanzenarten ausgewählt ist: *Copaifera officinalis, Copaifera multijugla* und *Copaifera reticulata;* die allein oder gemischt verwendet werden.

3. Extrakt zu seiner Verwendung nach einem der Ansprüche 1 oder 2, **dadurch gekennzeichnet, dass** er, bezogen auf das Gesamtgewicht des Extrakts, zwischen 7,5 und 95 Gew.-% Diterpensäuren und/oder Diterpensäureester enthält.

4. Extrakt zu seiner Verwendung nach einem der Ansprüche 1 bis 3, **dadurch gekennzeichnet, dass** das Gemisch aus Diterpensäuren und/oder Diterpensäureestern die folgenden Diterpensäuren und/oder Diterpensäureester umfasst: Copalsäure, Copaiferolsäure, Dimethylester der Agathendiosäure, Agathsäure, Methylester der 3beta-Hydroxyanticopalsäure, Hardwickiinsäure, 7-alpha-Acetoxyhardwickiinsäure.

5. Dermatologische Zusammensetzung, die einen Copaifera-Extrakt nach einem der Ansprüche 1 bis 4 mit mindestens einem dermatologisch akzeptablen Trägerstoff zu ihrer Verwendung bei der Behandlung von Alopezie umfasst.

6. Zusammensetzung zu ihrer Verwendung nach Anspruch 5, **dadurch gekennzeichnet, dass** der Copaifera-Extrakt der einzige gegen Alopezie wirkende Wirkstoff der Zusammensetzung ist.

7. Zusammensetzung zu ihrer Verwendung nach Anspruch 5 bis 6, **dadurch gekennzeichnet, dass** die Menge an Copaifera-Extrakt zwischen 0,05 % bis 10 Gew.-% im Verhältnis zum Gesamtgewicht der Zusammensetzung beträgt.

8. Zusammensetzung nach einem der Ansprüche 5 oder 6 zu ihrer Verwendung zur Verlangsamung des Haarausfalls.

9. Zusammensetzung zu ihrer Verwendung nach einem der Ansprüche 5 bis 8 in einer für eine topische Anwendung geeigneten Form.

## Claims

1. Copaifera extract comprising, as an active ingredient inhibiting 5-alpha reductase, a mixture of diterpene acids and/or esters of diterpene acids, for use in the prevention and/or treatment of alopecia, **characterised in that** the extract consists of the nonvolatile fraction of Copaifera oleoresin and that the mixture of diterpene acids and/or esters of diterpene acids comprises at least 6, or even at least 7, diterpene acids and/or esters of diterpene acids selected from the group consisting of: copalic acid, copaiferolic acid, dimethyl ester of agathendioic acid, agathic acid, methyl ester of 3B-hydroxyanticopaliic acid, hardwickiic acid, 7α-acetoxyhardwickiic acid.

2. An extract for use according to claim 1, **characterised in that** the *Copaifera* is selected from the following plant species: *Copaifera officinalis, Copaifera multijugla* and *Copaifera reticulata;* used alone or in combination.

3. Extract for use according to one of claims 1 or 2, **characterised in that** it contains, by weight relative to the total weight of the extract, between 7.5 and 95% of diterpene acids and/or esters of diterpene acids.

4. Extract for use according to any one of claims 1 to 3, **characterised in that** the mixture of diterpene acids and/or diterpene acid esters comprises the following diterpene acids and/or diterpene acid esters: copalic acid, copaiferolic acid, dimethyl ester of agathendioic acid, agathic acid, methyl ester of 3B-hydroxyanticopalic acid, hardwickiic acid, 7α-acetoxyhardwickiic acid.

5. A dermatological composition comprising a Copaifera extract according to any one of claims 1 to 4, together with at least one dermatologically acceptable excipient, for use in the treatment of alopecia.

6. Composition for use according to claim 5, **characterised in that** the Copaifera extract is the sole anti-alopecia active ingredient of said composition.

7. Composition for use according to claim 5 or 6, **characterised in that** the amount of Copaifera extract is between 0.05% and 10% by weight relative to the total weight of the composition.

8. A composition according to one of claims 5 or 6, for use in slowing hair loss.

9. Composition for use according to one of claims 5 to 8, in a form suitable for topical administration.
